# EUROPEAN PATENT SPECIFICATION

(11) **EP 1 512 754 B1**
(45) Date of publication and mention of the grant of the patent: **07.10.2009**
(21) Application number: 04020604.7
(22) Date of filing: 31.08.2004
(51) Int. Cl.: C12Q 1/68

(54) **Detection reagent for thermostable direct hemolysin gene of vibrio parahaemolyticus**
Reagenz zum Nachweis des TDH Gens von Vibrio Parahaemolyticus
Réactif de détection pour le géne TDH de vibrio parahaemolyticus

(30) Priority: 05.09.2003 JP 2003314130
(43) Date of publication of application: 09.03.2005
(73) Proprietor: TOSOH CORPORATION, Shunan-shi Yamaguchi 746-8501 (JP)
(72) Inventor: Masuda, Noriyoshi, Tokyo, 153-0063 (JP); Horie, Ryuichi, Zama-shi Kanagawa, 228-0001 (JP); Yasukawa, Kiyoshi, Kawasaki-shi Kanagawa, 215-0021 (JP)
(74) Representative: Pautex Schneider, Nicole Véronique

(56) References cited:
- EP-A- 1 134 292
- ISHIGURO ET AL: "Flourescence detection of specific sequence of nucleic acids by oxazole yellow-lnked oligonucleotides. Homogenous quantitative monitoring of in vitro transcription" NUCLEIC ACIDS RESEARCH, OXFORD UNIVERSITY PRESS, SURREY, GB, vol. 24, no. 24, 1996, pages 4992-4997, XP002093536 ISSN: 0305-1048

## Description

### Field of the Invention

The present invention relates to a detection reagent for detecting *Vibrio parahaemolyticus* in clinical examinations, public health examinations, food evaluations and food poisoning examinations.

### Prior Art

*Vibrio parahaemolyticus* is commonly known as a causative organism of infectious food poisoning. In contrast to 95% or more of *Vibrio parahaemolyticus* isolated from gastroenteritis patients being Kanagawa phenomenon-positive bacteria demonstrating hemolytic activity in Wagatsuma medium, 99% of the bacteria isolated from fish and water are Kanagawa phenomenon-negative. Thus, there is considered to be a close relationship between pathogenic *Vibrio parahaemolyticus* and the Kanagawa phenomenon.

Subsequently, this Kanagawa phenomenon was determined to occur due to the release of the thermostable direct hemolysin (TDH) of *Vibrio parahaemolyticus* outside the bacterial cells, which led to increasing attention being focused on *Vibrio parahaemolyticus* as a pathogenic factor. The TDH gene is currently known to exist as five types of gene consisting of TDH1 to TDH5.

More recently, from a microbial strain that demonstrates pathogenicity despite being negative for the Kanagawa phenomenon, a hemolysin having a base sequence similar to TDH as well as having partial common antigenicity had been identified (TDH-related hemolysin [TRH]). The TRH gene is currently known to exist as two types consisting of TRH1 and TRH2.

Although methods for detecting and identifying *Vibrio parahaemolyticus* comprising evaluation for Kanagawa phenomenon following enrichment culturing or isolation culturing are known, methods which detect a specific sequence present in the *Vibrio parahaemolyticus* gene or an RNA derived from said gene following the amplification of such a sequence are preferable in terms of sensitivity, speed and ease of procedure. A method that amplifies a target nucleic acid at a constant temperature is particularly preferable in terms of automation of a testing system.

A method for detecting and identifying *Vibrio parahaemolyticus* has been reported in which an RNA derived from TDH gene is specifically amplified at a comparative low temperature (41°C) (Japanese Unexamined Patent Publication Nos. 2001-258570 and 2001-340086). In this method, an RNA amplification process is used in which a double-strand RNA-DNA is formed by producing a cDNA with an RNA-dependent DNA polymerase using a specific sequence of an RNA derived from said TDH gene as a template, as well as a first primer having a sequence complementary to said specific sequence, and a second primer having a sequence homologous to said specific sequence, wherein the first primer or second primer has a sequence in which a promoter sequence of an RNA polymerase is added to the 5' end of one of the primers, degrading the RNA of the double-strand RNA-DNA by ribonuclease H, thereby producing a single-strand DNA, and producing a double-strand DNA having the promoter sequence capable of transcribing the RNA composed of the RNA sequence or the sequence complementary to the RNA sequence with a DNA-dependent DNA polymerase using said single-strand DNA as a template, wherein said double-strand DNA produces an RNA transcription product in the presence of the RNA polymerase, and said RNA transcription product serves as a template for the subsequent cDNA synthesis with the RNA-dependent DNA polymerase. In a preferable embodiment, the RNA amplification process is carried out in the presence of an oligonucleotide that has been labeled with an intercalator fluorescent pigment, and the detection is carried out by measuring the fluorescent intensity of the reaction solution; wherein, the sequence of said oligonucleotide is complementary to at least a portion of the sequence of the RNA transcription product and, in the situation where complementary binding of said oligonucleotide to said RNA transcription product occurs, the fluorescent properties of the reaction solution change in comparison with the situation where no complex is formed.

However, the aforementioned method has problems in terms of its sensitivity and speed. Japanese Unexamined Patent Publication No. 2001-340086 describes that when TDH RNA is detected in an initial RNA amount of 10³ copies, the increasing rate of the fluorescent intensity ratio with time is significantly slower that than when the initial amount is 10⁴ copies. As a result, even with a reaction time of 30 minutes, the fluorescent intensity ratio is 2.0 at the most. Further, even when the initial RNA amount is 0 copies, the fluorescent intensity ratio tends to increase, and as a result, when detecting TDH RNA at an initial RNA amount of 10² copies, even with a reaction time of 30 minutes, a significant increase of the fluorescent intensity ratio cannot be observed.

Therefore, the object of the present invention is to provide a detection reagent for TDH RNA that has superior sensitivity and speed.

### Disclosure of the Invention

As a result of extensive studies to develop a detection reagent for the thermostable direct hemolysin (TDH) RNA of *Vibrio parahaemolyticus* having a superior sensitivity and a higher speed, the inventors of the present invention developed a reagent capable of detecting TDH, even at an initial RNA amount of 10² copies, within a period of 20 minutes.

The present invention relates to a detection reagent for use in detecting the TDH gene of *Vibrio parahaemolyticus,* present in a sample that is used in a detection method using an RNA amplification process, comprising the steps of:
producing a cDNA with an RNA-dependent DNA polymerase using a specific sequence of an RNA derived from said TDH gene, that is, at least a partial sequence of said RNA, as a template, as well as a first primer having a sequence complementary to said specific sequence, and a second primer having a sequence homologous to said specific sequence, thereby forming a double-strand RNA-DNA ;
degrading the RNA portion of said double-strand RNA-DNA by ribonuclease H, thereby producing a single-strand DNA; and
producing a double-strand DNA having said promoter sequence capable of transcribing the RNA composed of the specific sequence of the RNA or the sequence complementary to said specific sequence of the RNA with a DNA-dependent DNA polymerase using said single-strand DNA as a template; wherein,
the double-strand DNA produces an RNA transcription product in the presence of the RNA polymerase, and said RNA transcription product serves as a template for the subsequent cDNA synthesis with the RNA-dependent DNA polymerase;
which reagent comprises,
as the first primer, an oligonucleotide consisting of the sequence listed as SEQ. ID No. 2, SEQ. ID No. 10 or SEQ. ID No. 11; and
as the second primer, an oligonucleotide consisting of the sequence listed as SEQ. ID No. 7, SEQ. ID No. 8 or SEQ. ID No. 9.

Furthermore, in the case of intending to detect an RNA complementary to the RNA derived from the TDH gene, an oligonucleotide having a sequence complementary to the aforementioned first primer with its sequence from the 5' end to the 3' end being reversed should be used as the first primer, an oligonucleotide having a sequence complementary to the aforementioned second primer with its sequence from the 5' end to the 3' end being reversed should be used as the second primer.

Preferably, the aforementioned RNA amplification process is carried out in the presence of a cleaving oligonucleotide that cleaves the aforementioned target RNA at the 5' end of the aforementioned specific sequence and has a sequence complementary to the region adjacent to and overlapping with the 5' end of said specific sequence. Said oligonucleotide is preferably an oligonucleotide consisting of the sequence listed as SEQ. ID No. 4, 5 or 6.

More preferably, the aforementioned RNA amplification process is carried out in the presence of an oligonucleotide labeled with an intercalator fluorescent pigment, and the detection of the thermostable direct hemolysin of *Vibrio parahaemolyticus* is carried out by measuring the fluorescent intensity of the reaction solution. Here, the sequence of said oligonucleotide is complementary to at least a portion of the sequence of the mRNA transcription product, and in the situation where complementary binding of said oligonucleotide to said RNA transcription product occurs, the fluorescent properties of the reaction solution change in comparison with the situation where no complex is formed.

Preferably, the aforementioned oligonucleotide consists of at least 10 contiguous bases in any of the sequences listed as SEQ. ID No. 3.

The following provides a detailed explanation of the present invention.

### Brief Description of the Drawings

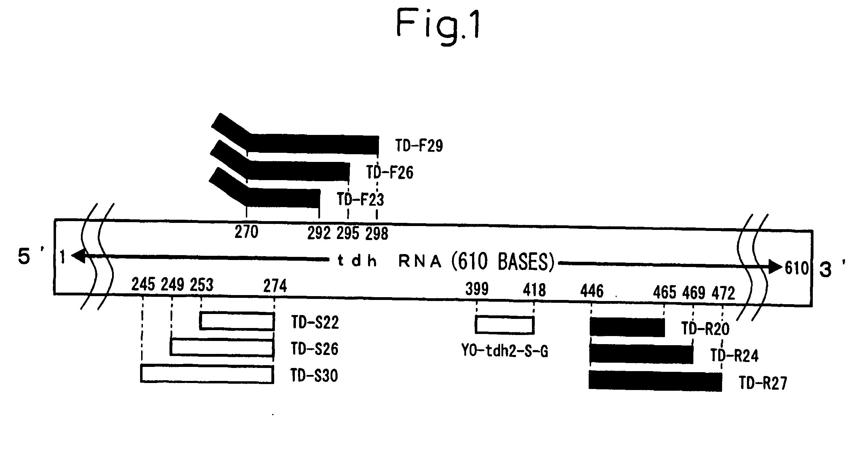

Fig. 1 shows the location of each oligonucleotide used in Example 1 along with the amplified regions. The base numbers in the drawing are in accordance with the literature (Appl. Environ. Microbiol., 58, 2449-2457 (1992)).

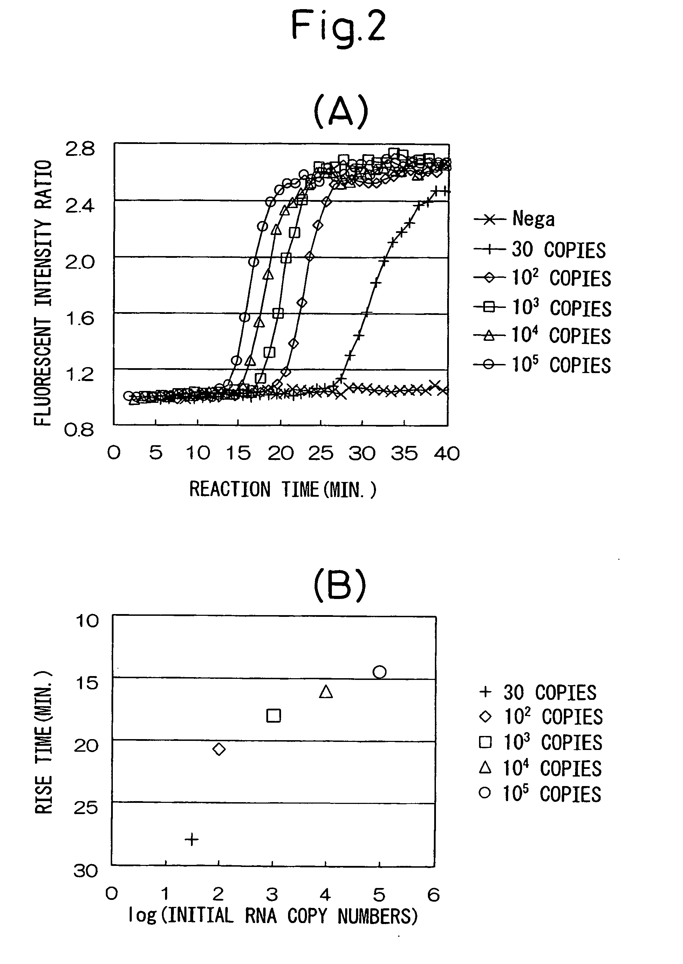

Fig. 2 (A) shows a graph of the fluorescent intensity ratio that increases with the reaction time and the production of RNA from an initial TDH RNA amount of 30 copies/test to 10⁵ copies/test carried out in Example 2, and (B) shows a calibration curve obtained between the logarithmic value of the initial RNA amount and the rise time. "Nega" refers to a sample in which a diluent was used instead of an RNA sample. TDH RNA for which the initial RNA amount was 10² copies/test could be detected after a reaction time of about 20 minutes, and a correlation was observed between the initial RNA amount and the rise time.

### Best Mode for Carrying Out the Invention

The following provides a detailed explanation of the present invention.

In the present invention, although the entire length of the base sequences listed in each of the sequence listings can be used for the first and second primers, respectively, as about 10 bases are sufficient for specific binding to a specific nucleic acid sequence or the like, a combination of at least 10 contiguous bases in each sequence may also be used.

The amplification process of the present invention includes the NASBA method, 3SR method or, for example, the RNA detection method (TRC method) described in Japanese Unexamined Patent Publication No. 2000-014400, which amplifies TDH RNA by concerted action of reverse transcriptase and RNA polymerase (by reacting them under a condition where the reverse transcriptase and RNA polymerase act in concert). Here, although there are no particular limitations on temperature, it is preferably 35 to 50°C.

In one aspect of the aforementioned invention of the present application, it is necessary for a target RNA to be cleaved at the 5' end of a specific sequence. A preferable method for cleaving the target RNA in this manner preferably consists of cleaving the target RNA with ribonuclease H or the like by adding an oligonucleotide having a sequence complementary to the region adjacent to and overlapping with the 5' end of the specific sequence (cleaving oligonucleotide). Said oligonucleotide is preferably an oligonucleotide consisting of a sequence listed as SEQ. ID No. 4, 5 or 6. In said cleaving oligonucleotide, the 3' end hydroxyl group is preferably chemically modified, for example, aminated, in order to suppress an elongation reaction from the 3' end.

Although the amplification product obtained in the aforementioned nucleic acid amplification method can be detected with a known nucleic acid detection method, in a preferable aspect of this method, the aforementioned nucleic acid amplification is preferably carried out in the presence of an oligonucleotide labeled with an intercalator fluorescent pigment followed by measurement of the change in the fluorescent properties of the reaction solution. In said oligonucleotide, as the intercalator fluorescent pigment is bound to the phosphorous atom in the oligonucleotide by means of a linker, the intercalator portion that forms a double strand with the target nucleic acid (complementary nucleic acid) intercalates to the double strand portion resulting in a change in fluorescent properties, thereby resulting in the characteristic of not requiring separation and analysis (Ishiguro, T. et al. (1996) Nucleic Acid Res. 24 (24) 4992-4997).

The sequence bound by said oligonucleotide may be any sequence specific for TDH RNA, and although there are no particular limitations thereon, a sequence consisting of at least 10 contiguous bases in the sequence listed as SEQ. ID No. 3 or its complementary sequence is preferable. In addition, the hydroxyl group at the 3' end of said oligonucleotide is preferably chemically modified (such as by an addition of glycolic acid) to suppress the elongation reaction which may occur by using this oligonucleotide as a primer.

As a result, TDH RNA of *Vibrio parahaemolyticus* can be amplified and detected in a single tube, at a constant temperature and in a single step, rapidly and with high sensitivity, thereby facilitating application to automation.

Although the following provides a more detailed explanation of the invention of the present application through examples, the present invention is not limited by these examples.

### Examples

### Example 1

The amplification efficiency of TDH RNA of *Vibrio parahaemolyticus* was compared for combinations (a) through (i) shown in Table 1.
(1) A sample of a standard RNA (616 bases) comprising base numbers 1 through 610 of TDH2 RNA of *Vibrio parahaemolyticus* (the base numbering of the RNA are in accordance with Nishibuchi, et al., Appl. Environ. Microbiol., 58, 2449-2457 (1992)) was quantified by ultraviolet absorption at 260 nm, and then diluted with an RNA diluent (10 mM Tris-HCl buffer (pH 8.0), 1 mM EDTA, 5 mM DTT, 0.5 U/µL RNase inhibitor (Takara Bio)) to 10³ copies/5 µL and 10⁴ copies/5 µL. Only diluent was used for the control group (negative control).
(2) 20 µL of a reaction solution having the composition indicated below was dispensed into 0.5 mL PCR tubes (GeneAmp Thin-Walled Reaction Tubes, Applied Biosystems) followed by the addition of 5 µL of the aforementioned RNA sample thereto. Furthermore, solutions were prepared so that the combinations of the first primer, the second primer and the cleaving oligonucleotide were combined as shown in Table 1.
Composition of Reaction Solution
(concentrations are shown as the concentration in the final reaction solution volume of 30 µL)
60 mM Tris-HCl buffer (pH 8.6)
17 mM magnesium chloride
110 mM potassium chloride
6 U RNase inhibitor
1 mM DTT
0.25 mM each of dATP, dCTP, dGTP and dTTP
3.6 mM ITP
3.0 mM each of ATP, CTP, GTP and UTP
0.16 µM cleaving oligonucleotide
1.0 µM second primer
1.0 µM first primer
15 nM oligonucleotide labeled with intercalator pigment (YO-TDH2-S-G, SEQ ID. No. 3; labeled with the intercalator fluorescent pigment between the 16th "G" and 17th "A" from the 5' end, and the hydroxyl group on its 3' end being modified with a glycol group.)
13% DMSO
Distilled water for adjusting volume
(3) After incubating the aforementioned reaction solution at 44°C for 5 minutes, 5 µL of an enzyme solution having the composition indicated below and preheated for 2 minutes at 44°C was added.
Composition of Enzyme Solution (values shown indicate the values for a final reaction solution volume of 30 µL)
2.0% sorbitol
3.6 µg bovine serum albumin
142 U T7 RNA polymerase (Invitrogen)
6.4 U AMV reverse transcriptase (Takara Bio)
Distilled water for adjusting volume
(4) Subsequently, the reaction solution in each of the PCR tubes was measured, over time, at an excitation wavelength of 470 nm and fluorescent wavelength of 520 nm, while being incubated at 44°C, using a fluorescent spectrophotometer equipped with a temperature control function and capable of directly measuring the tube.
(5) The "rise time" result (the time required for the ratio in the fluorescence increase to reach 1.2 times the sum of the negative control sample's average value plus 3 standard deviations) obtained by using each oligonucleotide combination is shown in Table 2. As TDH2 RNA was detected within 30 minutes regardless of which combination (a) through (i) was used, the oligonucleotides used in these combinations were shown to be effective for detecting TDH RNA of *Vibrio parahaemolyticus.*

**Table 1**

| Combination | Cleaving Oligo. | Second primer | First primer | Amplification product length (bases) |
|---|---|---|---|---|
| (a) | TD-S22 | TD-F23 | TD-R20 | 202 |
| (b) | TD-S22 | TD-F23 | TD-R24 | 206 |
| (c) | TD-S22 | TD-F23 | TD-R27 | 209 |
| (d) | TD-S26 | TD-F26 | TD-R20 | 202 |
| (e) | TD-S26 | TD-F26 | TD-R24 | 206 |
| (f) | TD-S26 | TD-F26 | TD-R27 | 209 |
| (g) | TD-S30 | TD-F29 | TD-R20 | 202 |
| (h) | TD-S30 | TD-F29 | TD-R24 | 206 |
| (i) | TD-S30 | TD-F29 | TD-R27 | 209 |

Table 1 shows the combinations of the first primer, the second primer and the cleaving oligonucleotide used in the experimental system along with the lengths of the specific bands amplified using those combinations. The locations and amplified regions of the oligonucleotides in TDH RNA of *Vibrio parahaemolyticus* are shown for each of the oligonucleotide combinations in Fig. 1. The hydroxyl group on the 3' end of the base sequence of the cleaving oligonucleotide was aminated. The region from the 1st "A" to the 22nd "A" from the 5' end of the base sequence of the second primer is a T7 promoter region, and the subsequent region from the 23rd "G" to the 28th "A" is an enhancer sequence.
Cleaving oligonucleotides:
TD-S22 (SEQ. ID No. 4, base nos. 253 to 274)
TD-S26 (SEQ. ID No. 5, base nos. 249 to 274)
TD-S30 (SEQ. ID No. 6, base nos. 245 to 274)
Second primers:
TD-F23 (SEQ. ID No. 7, base nos. 270 to 292)
TD-F26 (SEQ. ID No. 8, base nos. 270 to 295)
TD-F29 (SEQ. ID No. 9, base nos. 270 to 298)
First primers:
TD-R20 (SEQ. ID No. 10, base nos. 446 to 465)
TD-R24 (SEQ. ID No. 11, base nos. 446 to 469)
TD-R27 (SEQ. ID No. 2, base nos. 446 to 472)

**Table 2**

| Combination | Rise time (min) | | | |
|---|---|---|---|---|
| | 10³ copies/test | | 10⁴ copies/test | |
| (a) | 16.9 | 16.5 | 14.8 | 15.2 |
| (b) | 17.2 | 15.8 | 14.4 | 14.6 |
| (c) | 15.5 | 16.4 | 13.8 | 14.7 |
| (d) | 19.1 | 18.7 | 16.1 | 17.1 |
| (e) | 17.5 | 18.1 | 15.4 | 15.2 |
| (f) | 17.3 | 18.2 | 15.5 | 16.2 |
| (g) | 19.1 | 19.4 | 17.9 | 18.0 |
| (h) | 18.2 | 21.2 | 16.6 | 16.6 |
| (i) | 19.3 | 19.5 | 17.6 | 16.9 |

Table 2 shows the results of measuring TDH2 RNA at 10³ and 10⁴ copies/test using the combinations of oligonucleotides shown in Table 1. All of the combinations of oligonucleotides shown in Table 1 detected TDH2 RNA within 30 minutes.

### Example 2

Various initial numbers of copies of TDH2 DNA of *Vibrio parahaemolyticus* were detected using combination (a) shown in Table 1.
(1) TDH2 RNA of *Vibrio parahaemolyticus* similar to that of Example 1 were diluted to 10⁵ copies/5 µL to 30 copies/5 µL with an RNA diluent (10 mM Tris-HCl (pH 8.0), 1 mM EDTA, 5 mM DTT, 0.5 U/µL RNase inhibitor (Takara Bio)). Only diluent was used for the control group (negative control).
(2) 20 µL of a reaction solution having the composition indicated below was dispensed into PCR tubes (volume: 0.5 mL, GeneAmp Thin-Walled Reaction Tubes, Applied Biosystems) followed by the addition of 5 µL of the aforementioned RNA sample thereto.
   Composition of Reaction Solution (concentrations are shown as the concentration in the final reaction solution volume of 30 µL)
   60 mM Tris-HCl buffer (pH 8.6)
   17 mM magnesium chloride
   110 mM potassium chloride
   6 U RNase inhibitor
   1 mM DTT
   0.25 mM each of dATP, dCTP, dGTP and dTTP
   3.6 mM ITP
   3.0 mM each of ATP, CTP, GTP and UTP
   0.16 µM cleaving oligonucleotide (TD-S22, SEQ. ID No. 4, hydroxyl group of its 3' end is aminated)
   1.0 µM second primer (TD-F23, SEQ. ID No. 7)
   1.0 µM first primer (TD-R20, SEQ. ID No. 10)
   15 nM oligonucleotide labeled with intercalator pigment (YO-TDH2-S-G, SEQ ID. No. 3, labeled with the intercalator fluorescent pigment between the 16th "G" and 17th "A" from the 5' end, and the hydroxyl group on its 3' end being modified with a glycol group.)
   13% DMSO
   Distilled water for adjusting volume
(3) After incubating the aforementioned reaction solution at 44°C for 5 minutes, 5 µL of an enzyme solution having the composition indicated below and preheated for 2 minutes at 44°C were added.
   Composition of Enzyme Solution (values shown indicate the values for a final reaction solution volume of 30 µL)
   2.0% sorbitol
   3.6 µg bovine serum albumin
   142 U T7 RNA polymerase (Invitrogen)
   6.4 U AMV reverse transcriptase (Takara Bio)
   Distilled water for adjusting volume
(4) Subsequently, the reaction solution in each of the PCR tubes was measured, over time, at an excitation wavelength of 470 nm and fluorescent wavelength of 520 nm, while being incubated at 44°C, using a fluorescent spectrophotometer equipped with a temperature control function and capable of directly measuring the tube.
   By setting the time of the addition of the enzyme as 0 min., the time-dependent changes in the fluorescent intensity ratio of the samples (fluorescent intensity value at predetermined time ÷ background fluorescent intensity value) are shown in Figure 2(A).
   In addition, the results obtained for the relationship between the logarithmic value of the initial RNA amount and the "rise time" (the time required for the ratio in the fluorescence increase to reach 1.2 times the sum of the negative control sample's average value plus 3 standard deviations) are shown in Figure 2(B). Furthermore, the initial RNA amount ranged from 30 copies/test to 10⁵ copies/test.
   According to Fig. 2, 10² copies were detected in about 20 minutes. In addition, a fluorescent profile and a calibration curve depending on the initial target RNA amount were obtained, indicating the possibility of a quantitative detection of TDH RNA in an unknown sample. Moreover, as the present combination detected TDH2 even in an initial amount of 10² copies/test within about 20 minutes, it is possible to carry out detection of TDH more rapidly and with a higher sensitivity compared with the method of the prior art (Japanese Unexamined Patent Publications No. 2001-340086).

### Industrial Applicability

As has been explained above, the detection method of the invention of the present application is useful for detecting TDH RNA of *Vibrio parahaemolyticus* rapidly and with high sensitivity.

## Claims

1. A detection reagent for use in detecting the thermostable direct hemolysin (TDH) gene of *Vibrio parahaemolyticus* present in a sample that is used in a detection method using an RNA amplification process comprising the steps of:
producing a cDNA with an RNA-dependent DNA polymerase using a specific sequence of an RNA derived from said TDH gene as a template, as well as a first primer having a sequence complementary to said specific sequence, and a second primer having a sequence homologous to said specific sequence, thereby forming a double-strand RNA-DNA ;
degrading the RNA portion of said double-strand RNA-DNA by ribonuclease H, thereby producing a single-strand DNA; and
producing a double-strand DNA having said promoter sequence capable of transcribing the RNA composed of the specific sequence of the RNA or the sequence complementary to said specific sequence of the RNA with a DNA-dependent DNA polymerase using said single-strand DNA as a template; wherein,
the double-strand DNA produces an RNA transcription product in the presence of the RNA polymerase, and said RNA transcription product serves as a template for the subsequent cDNA synthesis with the RNA-dependent DNA polymerase;
which reagent comprises,
as the first primer, an oligonucleotide consisting of the sequence listed as SEQ. ID No. 2, SEQ. ID No. 10 or SEQ. ID No. 11; and
as the second primer, an oligonucleotide consisting of the sequence listed as SEQ. ID No. 7, SEQ. ID No. 8 or SEQ. ID No. 9.

2. The detection reagent according to claim 1 wherein the RNA amplification process is carried out in the presence of a cleaving oligonucleotide that cleaves the target RNA at the 5' end of the specific sequence and has a sequence complementary to the region adjacent to and overlapping with the 5' end of the specific sequence; wherein said oligonucleotide is an oligonucleotide consisting of the sequence listed as SEQ. ID No. 4, 5 or 6.

3. The detection reagent according to claim 1 or 2 wherein the RNA amplification process is carried out in the presence of an oligonucleotide that has been labeled with an intercalator fluorescent pigment, and the detection of the thermostable direct hemolysin of *Vibrio parahaemolyticus* is carried out by measuring the fluorescent intensity of the reaction solution; wherein the sequence of said oligonucleotide is complementary to at least a portion of the sequence of the mRNA transcription product, and in the situation where complementary binding of said oligonucleotide to said RNA transcription product occurs, the fluorescent properties of the reaction solution change in comparison with the situation where no complex is formed.

4. The detection reagent according to claim 3 wherein the oligonucleotide labeled with the intercalator fluorescent pigment consists of at least 10 contiguous bases of the sequence listed as SEQ. ID No. 3.

## Patentansprüche

1. Detektionsreagenz zur Verwendung beim Detektieren des in einer Probe vorhandenen thermostabilen direkten Hämolysin-(TDH-)Gens von *Vibrio parahaemolyticus,* das in einem Detektionsverfahren unter Anwendung eines RNA-Amplifikationsvorgangs verwendet wird, welches die Stufen:
- Produktion einer cDNA mit einer RNA-abhängigen DNA-Polymerase unter Verwendung einer speziellen Sequenz einer von diesem TDH-Gen abgeleiteten RNA als Template sowie eines ersten Primers mit einer Sequenz, die zu dieser speziellen Sequenz komplementär ist, und eines zweiten Primers mit einer Sequenz, die zu dieser speziellen Sequenz homolog ist, wodurch sich eine doppelsträngige RNA-DNA bildet,
- Abbau des RNA-Teils der doppelsträngigen RNA-DNA durch Ribonuclease H, wodurch eine einsträngige DNA produziert wird, und
- Produktion einer doppelsträngigen DNA mit der Promotorsequenz, die in der Lage ist, die RNA, die sich aus der speziellen RNA-Sequenz oder der zu dieser speziellen RNA-Sequenz komplementären Sequenz zusammensetzt, mit einer DNA-abhängigen DNA-Polymerase unter Verwendung der einsträngigen DNA als Template zu transkribieren, umfasst, wobei
- die doppelsträngige DNA ein RNA-Transkriptionsprodukt in Gegenwart der RNA-Polymerase produziert und dieses RNA-Transkriptionsprodukt als Template für die anschließende cDNA-Synthese mit der RNA-abhängigen DNA-Polymerase dient,
wobei das Reagenz
- als ersten Primer ein Oligonucleotid, das aus der Sequenz, die als SEQ ID Nr. 2, SEQ ID Nr. 10 oder SEQ ID Nr. 11 aufgezählt ist, besteht, und
- als zweiten Primer ein Oligonucleotid, das aus der Sequenz, die als SEQ ID Nr. 7, SEQ ID Nr. 8 oder SEQ ID Nr. 9 aufgezählt ist, besteht,
umfasst.

2. Detektionsreagenz nach Anspruch 1, wobei der RNA-Amplifikationsvorgang in Gegenwart eines spaltenden Oligonucleotids durchgeführt wird, das die Target-RNA am 5'-Ende der speziellen Sequenz spaltet und eine Sequenz besitzt, die zu der Region, die an das 5'-Ende der speziellen Sequenz angrenzt und mit diesem überlappt, komplementär ist, wobei dieses Oligonucleotid ein solches ist, das aus einer Sequenz besteht, die als SEQ ID Nr. 4, 5 oder 6 aufgezählt ist.

3. Detektionsreagenz nach Anspruch 1 oder 2, wobei der RNA-Amplifikationsvorgang in Gegenwart eines Oligonucleotids durchgeführt wird, das mit einem interkalierten Fluoreszenzpigment markiert worden ist, und die Detektion des thermostabilen direkten Hämolysins von *Vibrio parahaemolyticus* durch die Messung der Fluoreszenzintensität der Reaktionslösung durchgeführt wird, wobei die Sequenz des Oligonucleotids zu mindestens einem Teil der Sequenz des mRNA-Transkriptionsprodukts komplementär ist, und dann, wenn das komplementäre Binden dieses Oligonucleotids an dieses RNA-Transkriptionsprodukt stattfindet, sich die Fluoreszenzeigenschaften der Reaktionslösung im Vergleich mit dem Vorgang, bei welchem sich kein Komplex bildet, verändern.

4. Detektionsreagenz nach Anspruch 3, wobei das Oligonucleotid, das mit dem interkalierten Fluoreszenzpigment markiert ist, aus mindestens 10 aneinander angrenzenden Basen der als SEQ ID Nr. 3 aufgezählten Sequenz besteht.

## Revendications

1. Réactif de détection pour une utilisation dans la détection du gène de l'hémolysine thermostable directe (TDH) de *Vibrio parahaemolyticus* présent dans un échantillon qui est utilisé dans un procédé de détection utilisant un procédé d'amplification d'ARN comprenant les étapes de :
production d'un ADNc avec une ADN polymérase dépendante de l'ARN utilisant une séquence spécifique d'un ARN dérivé dudit gène de TDH comme matrice, ainsi qu'une première amorce ayant une séquence complémentaire de ladite séquence spécifique, et une deuxième amorce ayant une séquence homologue à ladite séquence spécifique, formant par ce moyen un double brin ARN-ADN ;
dégradation de la partie ARN dudit double brin ARN-ADN par une ribonucléase H, produisant par ce moyen un ADN simple brin ; et
production d'un ADN double brin ayant ladite séquence promotrice capable de transcrire l'ARN composée de la séquence spécifique de l'ARN ou de la séquence complémentaire de ladite séquence spécifique de l'ARN avec une ADN polymérase dépendante de l'ADN utilisant ledit ADN simple brin comme matrice ; dans lequel,
l'ADN double brin produit un produit de transcription d'ARN en présence de l'ARN polymérase, et ledit produit de transcription d'ARN sert de matrice pour la synthèse ultérieure d'ADNc avec l'ADN polymérase dépendante de l'ARN ;
lequel réactif comprend,
comme première amorce, un oligonucléotide constitué de la séquence énoncée comme SEQ ID No. 2, SEQ ID No. 10 ou SEQ ID No. 11 ; et
comme deuxième amorce, un oligonucléotide constitué de la séquence énoncée comme SEQ ID No. 7, SEQ ID No. 8 ou SEQ ID No. 9.

2. Réactif de détection selon la revendication 1, dans lequel le procédé d'amplification d'ARN est réalisé en présence d'un oligonucléotide de clivage qui clive l'ARN cible au niveau de l'extrémité 5' de la séquence spécifique et possède une séquence complémentaire de la région voisine de et chevauchant l'extrémité 5' de la séquence spécifique ; dans lequel ledit oligonucléotide est un oligonucléotide constitué de la séquence énoncée comme SEQ ID No. 4, 5 ou 6.

3. Réactif de détection selon la revendication 1 ou 2, dans lequel le procédé d'amplification d'ARN est réalisé en présence d'un oligonucléotide qui a été marqué avec un pigment fluorescent intercalant, et la détection de l'hémolysine thermostable directe de *Vibrio parahaemolyticus* est réalisée en mesurant l'intensité de fluorescence de la solution réactionnelle ; dans lequel la séquence dudit oligonucléotide est complémentaire d'au moins une partie de la séquence du produit de transcription d'ARNm, et dans la situation où une liaison complémentaire dudit oligonucléotide audit produit de transcription d'ARN a lieu, les propriétés fluorescentes de la solution réactionnelle changent par rapport à la situation où aucun complexe n'est formé.

4. Réactif de détection selon la revendication 3, dans lequel l'oligonucléotide marqué avec le pigment fluorescent intercalant consiste en au moins 10 bases contigües de la séquence énoncée comme SEQ ID No. 3.


### SEQUENCE LISTING

<110> TOSOH Corporation
<120> Detection Reagent for Thermostable Direct Hemolysin Gene of Vibrio parahaemolyticus
<130> 1033907
<160> 11
<210> 1
<211> 29
<212> DNA
<213> Artificial Sequence
<220>
<223> TD-F29-T7
<400> 1
   ggtcaatcag tattcacaac gtcaggtac 29
<210> 2
<211> 27
<212> DNA
<213> Artificial Sequence
<220>
<223> TD-R27
<400> 2
   atagaatctt catcttcacc aacaaag 27
<210> 3
<211> 20
<212> DNA
<213> Artificial Sequence
<220>
<223> YO-TDH2-S-G
<400> 3
   agctgtactt gatctgattt 20
<210> 4
<211> 22
<212> DNA
<213> Artificial Sequence
<220>
<223> TD-S22
<400> 4
   tgaccataaa catctttgta cg 22
<210> 5
<211> 26
<212> DNA
<213> Artificial Sequence
<220>
<223> TD-S26
<400> 5
   tgaccataaa catctttgta cggttt 26
<210> 6
<211> 30
<212> DNA
<213> TD-S30
<220>
<223>
<400> 6
   tgaccataaa catctttgta cggttttctt 30
<210> 7
<211> 51
<212> DNA
<213> TD-F23
<220>
<223>
<400> 7
   aattctaata cgactcacta tagggagagg tcaatcagta ttcacaacgt c 51
<210> 8
<211> 54
<212> DNA
<213> TD-F26
<220>
<223>
<400> 8
   aattctaata cgactcacta tagggagagg tcaatcagta ttcacaacgt cagg 54
<210> 9
<211> 57
<212> DNA
<213> TD-F29
<220>
<223>
<400> 9
   aattotaata cgactcacta tagggagagg tcaatcagta ttcacaacgt caggtac 57
<210> 10
<211> 20
<212> DNA
<213> TD-R20
<220>
<223>
<400> 10
   cttcatcttc accaacaaag 20
<210> 11
<211> 24
<212> DNA
<213> TD-R24
<220>
<223>
<400> 11
   gaatcttcat cttcaccaac aaag 24